(19) Europäisches Patentamt
European Patent Office
Office européen des brevets

(11) **EP 4 675 631 A1**

(12) **EUROPEAN PATENT APPLICATION**

(43) Date of publication:
**07.01.2026 Bulletin 2026/02**

(21) Application number: **24185772.1**

(22) Date of filing: **01.07.2024**

(51) International Patent Classification (IPC):
**G16H 10/40** (2018.01)

(52) Cooperative Patent Classification (CPC):
**G16H 10/40**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(71) Applicant: **Beckman Coulter, Inc.**
**Brea, CA 92821 (US)**

(72) Inventors:
• **Mackowiak, Stephan A**
  **81249 Munich (DE)**
• **Linke, Marco**
  **80339 Munich (DE)**

(74) Representative: **Paustian & Partner**
**Patentanwälte mbB**
**Oberanger 32**
**80331 München (DE)**

(54) **A METHOD OF SELECTING A STORAGE UNIT FOR STORING A SAMPLE CONTAINER**

(57) The invention relates to a method comprising: selecting, by a computing device, based on distance data and occupancy data, a storage unit among a plurality of storage units for storing a sample container processed by an automated laboratory system.

The distance data specify a distance along a path for moving the sample container in the automated laboratory system, such that a biological sample included in the sample container is processed by the automated laboratory system according to a test order associated with the biological sample. The path comprises the storage unit. The occupancy data comprise information indicative of an occupancy of the storage unit.

Fig. 1

## Description

The present disclosure relates to a selecting a storage unit for storing a sample container processed by an automated laboratory system.

Typically, Automated Laboratory Systems (ALSs) are assemblies comprising a plurality of components which allow for processing (e.g., testing) biological samples contained in respective sample containers. Sample containers are typically used for holding samples to be tested, e.g., biological samples (for instance blood samples) drawn from a human or animal subject. An opening of the sample container may be closable, e.g. sealed, by a cap.

The components may for example include at least one of pre-processing components (e.g., an input region for inserting sample containers in the ALS, a decapper, a centrifuge, an aliquoter), a processing portion (e.g. for analysing a biological sample contained in the container) and a post-processing component (e.g., a storage unit for storing sample containers). Examples of transportation components comprise a track, a belt, and a carrier.

After feeding a sample container, e.g. via the pre-processing component, the sample container may be decapped by a decapper. An analytic instrument of a processing component may be configured to carry out one or more clinical tests using the sample. An analytical instrument may be a laboratory analyzer. After analyzing the sample contained in the sample container, the sample container may be recapped by a recapper and stored in a storage device.

A first aspect of the present disclosure refers to a method, comprising: selecting, by a computing device, based on distance data and occupancy data, a storage unit among a plurality of storage units (herein also referred to as: "first plurality of storage units") for storing a sample container (herein also referred to as: "first sample container") processed by an automated laboratory system, ALS.

The distance data specify a distance along a path (herein also referred to as: "selected path") for moving the sample container in the automated laboratory system, such that a biological sample included in the sample container is processed by the automated laboratory system according to a test order associated with the biological sample. The path comprises the storage unit. The occupancy data comprise information indicative of an occupancy of the storage unit.

By providing such a method, it becomes possible to increase the system throughput of the ALS or predict a system capacity more reliably. This is achieved by increasing the number of sample containers processed and stored per time unit, as explained in the following.

Typically, the ALS is fed with a plurality of sample containers, e.g. via an input module of the ALS. For instance, the input module may be configured to receive single sample containers or sample containers stored in a transportation rack. These sample containers are processed by the ALS at least partially simultaneously, as the processing time of a single sample container may be significantly longer than an average time interval between subsequently fed sample containers. As a consequence, there may be a plurality of sample containers under process in the ALS at the same time, i.e. simultaneously moving along respective paths (which may be identical or different to each other, depending on the test orders associated with the biological samples contained in the sample containers). Each of these sample containers under process needs to be stored after being processed in a storage unit. As long as the sample container is still under process, it increases the risk of a congestion in the ALS.

However, since according to the present disclosure the selection of the storage unit (for a particular sample container) depends on the distance data associated with the path along which the sample container moves, shorter paths can be privileged in the selection process, what may lead to shorter transport times and thus processing times. As a consequence, the total number of sample containers, which are simultaneously under process in the ALS, can be reduced. Therefore, congestions in the ALS can be avoided or at least reduced. Moreover, due to the shorter processing times and the reduction of congestions, the overall the number of sample containers processed and stored per time unit (i.e. "taken out" of the ALS) can be increased.

Furthermore, since the selection of a suitable storage unit for a particular sample container depends on the occupancy data, selection process can be dynamical. For example, in case a first storage unit is selected several times for several sample containers, its occupancy may change over time. Consequently, a second storage unit may become more suitable as destination for subsequent (future) sample containers. The method can thus allow to avoid congestions due to storage units having an overoccupancy, and to favorize other storage units in the selection process which have a relatively low occupancy.

Considering the distance data and the occupancy data has thus a synergistic effect, which allows preventing or reducing congestions in the ALS, thus increasing the system throughput of the ALS and predicting a system capacity of the ALS more reliably.

In particular, an automated laboratory system may be an assembly comprising a plurality of components. The ALS may comprise a computing device operatively connected to these components and is configured to control each component. A component of the ALS may be an analytic instrument, a pre-processing (e.g. pre-analytic) instrument, a post-processing (e.g. post-analytic) instrument, an input module, an output module, a transportation component (e.g., a track, a belt, a tube carrier and the like), configured to move a sample container. In the following, "processing component" and "instrument" may be used interchangeably.

In particular, the transportation components may allow for moving containers from one laboratory instrument to another laboratory instrument or for moving containers from one component of a laboratory instrument to another component of the same laboratory instrument or to another component of another laboratory instrument. The automation components may include one or more tracks, one or more belts and/or container carriers configured to move biological samples contained in sample containers.

An analytic instrument (hereinafter referred to also as "laboratory instrument") may be configured to carry out at least a clinical test. An analytical instrument may be a laboratory analyzer. The analytical instrument may include one or more pre-analysis, analysis, and post-analysis components.

An analyzer may be, in particular, a laboratory instrument configured to carry out one or more analytic steps, such as measuring one or more characteristics of a biological sample, e.g., the concentration of an analyte. The laboratory analyzer may include an immunoassay analyzer, a chemistry analyzer, and identification and antibiotic susceptibility analyzer, a bacteriology analyzer, a molecular analyzer, a hematology analyzer, a urinalysis instrument, and the like.

A pre-analysis laboratory instrument may be configured to carry out one or more analytic steps on a biological sample to prepare the biological sample for the analytic instrument(s). A pre-analysis laboratory instrument may include a centrifuge, a decapper, an input module and/or an aliquoter.

A post-analysis laboratory instrument may be configured to carry out one or more post-processing steps on the biological sample after the biological sample has been processed by one or more laboratory analyzers. A post-analysis laboratory instrument may include one or more of a recapper (e.g., for putting caps back on containers), a storage unit, in particular a refrigerated storage unit, and an output module.

Both pre-analysis and post-analysis laboratory instruments may be referred to as peri-analytical laboratory instruments.

A biological sample may be a sample of a bodily fluid of a human or animal subject. For example, the bodily fluid may be a physiological fluid, such as blood, saliva, urine, sweat, amniotic fluid, cerebrospinal fluid, ascites fluid, or the like. The biological sample may comprise one or more components that may potentially comprise analytes of interest for performing at least one clinical test. For instance, blood serum, blood cells and blood plasma are components of a blood sample.

Typically, the biological sample is put into a sample container. The sample container may be a sample tube. Typically, sample tubes comprise a closed tube end and an end opposite thereto. The latter end defines an opening for inserting the sample in the sample tube. The opening may be closeable, e.g. sealable, by a cap. In general, a sample container may be configured to receive and hold a biological sample. Accordingly, the sample container may comprise an opening for receiving the sample and a bottom as well as one or more walls to hold the sample, wherein the opening may be configured to be releasably closed by a closing element, e.g. a cap. In particular, the sample container may have a substantially cylindrical shape (i.e. a cylindrical wall), with the opening at one base and the bottom at the opposite base.

A test order associated with a biological sample may specify one or more clinical tests to be carried out on the biological sample. The test order associated with a biological sample may be comprised in a Laboratory Information System (LIS) together with information allowing for uniquely and unambiguously associate the test order with the corresponding biological sample. The LIS may be comprised by the ALS and/or by the computing device, may be external to the ALS and/or the computing device, and/or may be associated with the ALS and/or the computing device. For example, a LIS may be associated with a plurality of ALS. The LIS and the ALS may be communicatively connected with each other, e.g. for transmitting a test order from the LIS to the ALS. In particular, the method according to the present disclosure may comprise obtaining, by the computing device, the test order. Obtaining the test order may comprise accessing the test order, e.g., retrieving the test order from the LIS. Retrieving the test order may comprise downloading the test order. Additionally, or alternatively, accessing the test order may comprise receiving the test order, e.g. from the LIS. The two options are not mutually exclusive. For instance, accessing the test order may comprise receiving the test order from the LIS, storing the test order in the memory of the computer device and retrieving the test order by accessing said memory.

A clinical test may comprise one or more procedures that, when carried out on the biological sample or on a component thereof, allow for estimating the value of a parameter, e.g. a clinical parameter. In particular, a clinical test may comprise physical, biological, optical, mechanical, immunological, and/or chemical procedures.

Exemplarily, an automated laboratory system processes a biological sample included in a sample container by causing the sample container to travel within the automated laboratory system by using the transportation means of the automated laboratory system so that a subset of the instruments of the automated laboratory system can carry out the pre-analytical, analytical, and post-processing steps needed to perform the clinical tests in the test order associated with the biological sample.

In particular, a path for moving a sample container in the automated laboratory system, such that a biological sample included in the sample container is processed by the automated laboratory system according to the test order associated with the biological sample, may be a route that said sample container is caused to travel within the ALS so that the ALS may process the sample container according to the test order. Said route may comprise (e.g., may pass through) one or more analytic instruments, one or more pre-processing instruments, one or more post-processing instruments, and a plurality of transportation components that allows the sample container to travel in the analytic instruments, pre-processing instruments, and post-processing instruments comprised in said route. Alternatively, or additionally, the path comprises a plurality of transportation components that allows the sample container to travel in the vicinity of one or more pre-processing instruments, one or more post-processing instruments and one or more post-processing instruments that are not comprised in the route, so that said one or more pre-processing instruments, one or more post-processing instruments and one or more post-processing instruments are able to retrieve and process a portion of the biological sample contained in the sample container.

Determining a path for moving a sample container in the automated laboratory system, such that a biological sample included in the sample container is processed by the automated laboratory system according to the test order associated with the biological sample, may comprise obtaining the one or more clinical tests comprised in the test order, determining, e.g., by the computing device, the pre-processing steps (e.g., centrifugation, decapping, aliquoting, and the like) and post-processing steps (e.g., recapping, storing of the sample container and the like), if any, that shall be carried out to carry out the one or more clinical tests. In particular, determining said path may comprise determining the order according to which the pre-processing, the post-processing and the clinical tests shall be carried out to process the biological sample according to the test order. Determining said path may comprise determining the chronological order according to which the pre-processing, the clinical tests, and the post-processing shall be carried out to process the biological sample according to the test order. Determining a path comprises determining the analytic instruments, the pre-processing instruments and the post-processing instruments that shall process the biological sample and generate the path that allows said instruments to process the biological sample in the chronological order according to which the pre-processing, the post-processing and the clinical tests shall be carried out.

In particular, a path for moving a sample container in the automated laboratory system, such that a biological sample included in the sample container is processed by the automated laboratory system according to the test order associated with the biological sample comprises an input module of the one or more input modules of the ALS. For instance, an input module is configured to receive sample containers that are inputted in the ALS. For instance, input module may be or comprise a loading area (e.g. an inlet) of the automated laboratory system configured to receive single sample containers or sample containers stored in a transportation rack. An input module may also be or further comprise a distribution buffer, where sample containers may be positioned before being transferred on a transportation component, e.g., on a track or a belt, to be moved within the ALS.

The selected path comprises the storage unit, in particular, as an end point or final destination for the sample container. In particular, the selected path may be a route from an input module to the selected storage unit. In particular, the selected path may define the way the sample container is to be transferred through the automated laboratory system from the input module to the selected storage unit so that the biological sample included in the sample container is processed by the automated laboratory system according to the test order associated with the biological sample.

According to the present disclosure, the occupancy of a storage unit may be, e.g., a numerical value indicative of one or more of: the amount of sample containers stored in that storage unit and the amount of sample containers stored in that storage unit relative to the maximum capacity of said storage unit, i.e., relative to the maximum number of sample containers storable in that storage unit. The occupancy of a storage unit may be an occupancy ratio, occupancy degree or occupancy state of that storage unit. In particular, the occupancy ratio may be or may be specified by the number of the sample containers currently stored in the storage unit in relation to the maximum number of storable sample containers. The occupancy level may be or be specified by the number of sample containers, that are currently stored in the storage unit. The occupancy level be specified by two (integer) numbers, the first one being the number of sample containers, that are currently stored in the storage unit and the second one being the maximum number of sample containers storable in the storage unit. The occupancy state may comprise information specifying to which of a set of predefined classes the occupancy ratio or the occupancy level belong. For instance, the predefined classes may comprise the classes "substantially empty" (e.g., occupancy ratio between from 0% to 10%), "partially filled", (e.g., occupancy ratio between 11% and 89%) and "substantially full" (e.g., occupancy ratio between 90% and 100%) etc. For example, there may be several "partially filled" classes such as, "partially filled - level one", (e.g., occupancy ratio more than 10% and up to 20%") , "partially filled - level two" (e.g., occupancy ratio more than 20% and up to 40%), "partially filled - level three" (e.g., occupancy ratio more than 40% and up to 60%), "partially filled - level four" (e.g., occupancy ratio more than 60% and up to 80%), and "partially filled - level five" (e.g., occupancy ratio more than 80% and less than 90%).

The distance data may comprise or be a single value or a set of values. Similarly, the occupancy data may comprise or be a single value or a set of values. Examples are provided below.

Exemplarily, selecting the storage unit may comprise the automated process of determining the storage unit from the plurality of storage units. In particular, selecting the storage unit may comprise or be implemented by a set of computer readable instructions, e.g., stored in a memory of the computing device, which when executed by the computing device, cause the computing device to select, based on the distance data and the occupancy data, the storage unit among a plurality of storage units.

The selection process may be automated, i.e., it may be performed by the computing device without human intervention.

Furthermore, selecting the storage unit is based on the distance data and the occupancy data. Accordingly, selecting the storage unit may comprise evaluating one, several or all storage units of the plurality of storage units against predefined conditions or criteria and selecting the storage unit based on this evaluation.

For example, selecting the storage unit may comprise: obtaining the distance data and the occupancy data. Obtaining data may comprise accessing said data, e.g., retrieving said data from a computer memory or a database, e.g. from the memory of the computing device. Retrieving the data may comprise downloading the data. Additionally, or alternatively, accessing the data may comprise receiving the data, e.g. from another computing device or a database. For instance, accessing the test order may comprise receiving the data, storing the data in the memory of the computing device and retrieving the data by accessing said memory.

The method according to the present disclosure may comprise determining the selected path. In particular, determining the selected path may comprise selecting the storage unit and, optionally, determining a plurality of paths (e.g. "candidate paths"), each of the plurality of paths for moving the sample container in the ALS, such that the biological sample included in the sample container is processed by the automated laboratory system according to the test order. Each of the plurality of paths is associated with a particular storage unit among the plurality of storage units. In particular, the storage unit may be selected by selecting the path among the plurality of paths, e.g., by using one or more criteria. In this case, in particular, the selected storage unit is the storage unit comprised in the path.

The method according to the present disclosure may comprise determining, for each sample container of a plurality of sample containers, a respective path for said each sample container, wherein the plurality of sample containers comprises the first sample container and the respective path for the first sample container is the aforementioned selected path. In particular, determining, for each sample container of a plurality of sample containers, the respective path comprises determining the selected path. For instance, determining, for each sample container of a plurality of sample containers, the respective path may comprise selecting, for each sample container of a plurality of sample containers a respective storage unit, wherein the respective storage unit for the first sample container is the selected storage unit mentioned above. In particular, the selection of the respective storage units may be carried out as described herein with reference to the selected storage unit. Typically, an ALS may process thousand sample containers per hour. The method according to the present disclosure makes possible an automated determination of the paths for moving the plurality of sample container in the ALS.

Exemplarily, selecting the storage unit may comprise at least one of: determining a metric based on the distance data and the occupancy data, and determining whether the metric fulfills a first set of selection criteria. The metric may be associated with the storage unit. For example, each of the storage units may be associated with a metric.

For example, the first set of selection criteria may comprise one or more of: a criterium that the metric is lower than a first predetermined threshold, a criterium that the metric is lower than or equal to the first predetermined threshold, a criterium that the metric is greater than a second predetermined threshold, a criterium that the metric is greater than or equal to the second predetermined threshold.

Exemplarily, the metric, $M$, may for example be directly proportional to a length $L$, of the selected path. The metric $M$ may be inversely proportional to a (positive power of) an inoccupancy ratio, $I$. For example, the inoccupancy ratio, $I$, is in particular a numerical value equal to ratio between the number of available slots in the selected storage unit and the maximum capacity of said storage unit. In particular, the inoccupancy ratio, $I$, is equal to $(1 - R)$ wherein $R$ may be the occupancy ratio, defined as the number of sample containers stored in the selected storage unit relative to the maximum capacity of said storage unit, i.e., relative to the maximum number of sample containers storable in that storage unit.

In one example, the metric $M$ may be defined as:

$$M = \frac{L}{I^2} = \frac{L}{(1 - R)^2} \qquad (1).$$

For instance, the first set of selection criteria comprises or consists of the criterium that the metric, $M$, in equation (1) is lower than a predetermined threshold. For instance, the predetermined threshold may be comprised between 0.5 and 0.8, in particular between 0.5 and 0.7 and, more particularly, being equal to 0.5 or 0.6.

The distance along the path for moving the sample container may be specified by at least one of: a length of the path, a number of components of the automated laboratory system that are included in the path, and an estimate of a time needed to move the sample container along the path.

The automated laboratory system may comprise a plurality of components for processing the sample container which are at least indirectly connected with each other via one or more transportation means. In particular, the selected path may comprise a subset of said connected components and/or run in vicinity of another subset of said connected components from the input module to the selected storage unit.

The length of the path may be the total distance measured along the path between its start and end points, e.g. from an input module of the ALS to the (selected) storage unit. This can refer to the actual physical distance traversed along a specific route, whether it is straight or curved, and can be calculated by summing up the distances of each segment of the path, e.g. of each processing component and/or each transport component (as described in more detail below) comprised in the path.

The number of components may refer to the number of processing components comprised in the path and/or the number of transport components comprised in the path.

The estimate of the time needed to move the sample container along the path may represent an estimate of the time duration needed to move the sample container along the path from its start to its end point, e.g., from the input module of the ALS to the selected storage unit.

In particular, selecting the storage unit may comprise estimating the time needed to move the sample container along the path.

For example, the time may be estimated based on a predicted and/or an actual workload of the ALS and, in particular, on a prediction of the number of sample containers that are processed e.g. at the estimation of the time. In particular, the traffic may be predicted based on path data (as explained in more detail below) of other sample containers which are currently processed by the ALS. The path data of a particular sample container may specify an order according to which the sample container and/or the biological sample contained therein should be handled by the components of the automated laboratory system, i.e., a temporal sequence of processing steps. Accordingly, based on the path data of the sample containers under process, the traffic in the ALS may be predicted, e.g. also the traffic at a particular component of the ALS at a particular future time point. It may thus also be possible to predict an occupancy ratio of a storage unit at a future time point, e.g. when the sample container is estimated to arrive at the storage unit.

In particular, estimating the time needed to move the sample container along the path may comprise (i) obtaining an expected time for moving the sample container along the transportation means (e.g. the transportation components) comprised in the selected path, (ii) obtaining, for each instrument included in the selected path, a respective expected time for completion of the processing steps that said instrument shall carry out of the sample container and/or on the biological sample and (iii) obtaining, for each instrument that is not included in the selected path but is to carry out some processing step on the biological sample (e.g. in case the sample is at least partially extracted from the sample container for an analysis step), a respective expected time for retrieving a portion of the biological sample contained in the sample container. For example, estimating the time needed to move the sample container along the path may comprise summing together the expected times mentioned above.

For example, obtaining an expected time for carrying out an action (e.g., for moving a sample container, processing a sample container or a biological sample included therein, retrieving a portion of a biological sample) may comprise retrieving said expected time by accessing the memory of the computing device. Obtaining an expected time for carrying out an action (e.g., for moving a sample container, processing a sample container or a biological sample included therein, retrieving a portion of a biological sample) may additionally or alternatively comprise determining said time from a respective statistical distribution of measured times needed to carry out said action. For example, the expected time may be the average of the respective statistical distribution or the sum of said average and the standard deviation of said distribution.

The occupancy of the storage unit may be specified by a fill ratio of the storage unit. The fill ratio may be a measure of how much of the storage unit's available capacity is currently being utilized. The capacity (or maximum capacity) may correspond to predefined number of available slots in the storage unit, each slot being configured to accommodate a sample containers. the fill ratio is typically expressed as a percentage and is calculated by the ratio between the number of slots of the storage unit that are occupied by sample containers and the maximum capacity of the storage unit.

By taking the fill ratio into account when selecting a storage unit, it can be assured that no storage unit will be supplied with more sample containers than can be stored in the storage unit. Moreover, it is possible to prioritize storage units having a lower fill ratio in the selection. For example, when a storage unit has a fill ratio exceeding a predefined fill ratio threshold, the resulting metric may make the storage unit unfavorable in the selection process.

The storage unit may comprise or may be associated with a respective recapper for recapping the sample container.

The occupancy of the storage unit may be defined as and/or specify an occupancy ratio of the recapper. For example, the occupancy ratio may be determined based on the actual occupancy of the recapper relative to a predetermined maximum occupancy capacity of the recapper.

In particular, the metric, $M$, may be given by equation (1), wherein $L$ is a length of the selected path and the inoccupancy ratio, $I$, is in particular equal to $(1 - R)$ wherein $R$ may be the occupancy ratio of the recapper.

For example, the recapper may comprise or be arranged in the vicinity of a queue space for temporarily buffering up to a predefined maximum amount of sample containers that have to be re-capped by the recapper. The actual occupancy of the recapper may be or may be determined based on the actual number of sample containers in that queue space. The maximum occupancy of the recapper may be or may be based on the predefined maximum amount. In particular, the maximum occupancy may be equal to the 80% or the 70% of the predefined maximum amount. More particularly, if the predefined amount is equal to 27 sample containers, the maximum occupancy of the recapper may be equal to 21, which is the integer part of 80% of the predefined maximum amount.

For instance, the actual occupancy of the recapper may be determined by using the data provided by a first sample container detector and a second sample container detector. In particular, the first sample container detector is arranged with respect to the queue space such that said detector detects sample containers entering the queue space. The second sample container detector is arranged with respect to the recapper such that said detector detects sample containers that leave the recapper and/or are recapped by the recapper and, hence, are about to leave the recapper.

The storage unit may further comprise a storage space for storing a plurality of sample containers. The storage space may have several slots, each slot being configured to accommodate a sample container. The sample container may be recapped and then placed in a storage space of the storage unit, e.g., in a slot of the storage space. In particular, the storage space may be a rack.

The automated laboratory system may comprise or be associated with at least one decapper for decapping the sample container before the sample is processed. A decapping rate of the at least one decapper may be substantially equal to a recapping rate of a recapper associated with a storage unit for recapping the sample container.

Exemplarily, e.g., depending on the number of the decappers comprised in the ALS, , a total decapping rate of the ALS (i.e., the sum of the recapping rates of the decappers comprised in the ALS) may be higher than a recapping rate of a single recapper. However, since the method of the present disclosure allows a dynamic distribution of processed sample containers between different storage units (i.e. by selection of a storage unit among a plurality of storage units), a congestion of sample containers in the ALS can be avoided. It becomes thus possible to e.g. equip the ALS with two or more input modules, each having one decapper. Each of these decappers may have a decapping rate similar to the recapping rate of a recapper of a storage unit. Consequently, by dynamically selecting storage units according to the method of the present disclosure, the throughput (i.e. throughput volume capability) of the ALS can be increased.

The storage unit may comprise or may be associated with a respective robotic apparatus (e.g., a robotic arm) for moving sample containers in the storage unit e.g., from the recapper associated to said storage unit.

The occupancy of the storage unit may be defined as and/or may specify as an occupancy ratio of the respective robotic apparatus. For example, said occupancy ratio may be determined based on the actual occupancy of the robotic apparatus related to a predetermined maximum occupancy capacity of the robotic apparatus.

In particular, the metric, $M$, may be given by equation (1), wherein $L$ is a length of the selected path and the inoccupancy ratio, $I$, is in particular equal to $(1 - R)$ wherein $R$ may be the occupancy ratio of the robotic apparatus.

For example, the robotic apparatus may comprise or be arranged in the vicinity of a queue space for temporarily buffering up to a predefined maximum amount of sample containers that have to be moved by the robotic apparatus. The actual occupancy of the robotic apparatus may be or may be determined based on the actual number of sample containers in that queue space. The maximum occupancy of the robotic apparatus may be or may be based on the predefined maximum amount. In particular, the maximum occupancy may be equal to the 80% or the 70% of the predefined maximum amount. More particularly, if the predefined amount is equal to 27 sample containers, the maximum occupancy of the recapper may be equal to 21, which is the integer part of 80% of the predefined maximum amount.

The method of the present disclosure may for example allow a more efficient management of the ALS, e.g. for waste disposal during peak working hours. Typically, the same transport component of the ALS (e.g. a robot) may take care of wasting expired sample containers from the storage units and store processed sample containers in the storage units. Waste disposal may typically have a relatively low priority with regard to sample container storage. Hence, if a storage unit is at full load (i.e. at a maximum occupancy ratio) there may be no possibility to dispose expired sample containers. The method of the present disclosure may however allow waste disposal even during peak hours, e.g. by favorizing storage units with a relatively low occupancy ratio in the selection process, i.e. such that each storage unit still has some capacity for waste disposal.

Selecting the storage unit may comprise determining, for each storage unit of the plurality of storage units, and based on respective distance data and respective occupancy data, a respective metric associated with said each storage unit, thereby obtaining a plurality of metrics. The respective distance data may specify a respective distance along a respective path in the automated laboratory system. The respective path may comprise said each storage unit. The respective occupancy data may comprise information indicative of a respective occupancy of said each storage unit.

Selecting the storage unit may be based on the plurality of metrics. Selecting the storage unit may for example comprise comparing the plurality of metrics (e.g. the selection may be determined based on a comparison of the metrics).

Exemplarily, the metric, $M_i$, of a storage unit, $S_i$, of the plurality of storage units may for example be directly proportional to a length $L_i$, of the path associated with this storage unit, $S_i$. The metric, $M_i$ may be inversely proportional to a (positive power of) an inoccupancy ratio, $I_i$ of the storage unit $S_i$. For example, the inoccupancy ratio $I_i$ is a numerical value equal to ratio between the number of available slots in the storage unit $S_i$ and the maximum capacity of said storage unit. In particular, the inoccupancy ratio $I_i$ is equal to $(1 - R_i)$ wherein $R_i$ is the occupancy ratio of the storage unit $S_i$, defined as the number of sample containers stored in the storage unit $S_i$ relative to the maximum capacity of said storage unit, e.g., relative to the maximum number of sample containers storable in that storage unit.

In particular, for each storage unit, $S_i$, of the first plurality of storage units, the respective Metric $M_i$ may be defined as:

$$ M_i = \frac{L_i}{I_i^2} = \frac{L_i}{(1 - R_i)^2} \qquad (2) $$

wherein for each storage unit, $S_i$, of the first plurality of storage units, $L_i$ is a length of the respective path in the automated laboratory system associated with that storage unit $S_i$. For each storage unit $S_i$ of the first plurality of storage units, $I_i$ is the inoccupancy ratio of that storage unit $S_i$. In particular, for each storage unit $S_i$ of the first plurality of storage units, $I_i$ is equal to $(1 - R_i)$ wherein $R_i$ may be the occupancy ratio associated with said storage unit $S_i$. For instance, for each storage unit $S_i$ of the first plurality of storage units, the occupancy ratio associated with said storage unit $S_i$, is the number of sample containers stored in that storage unit relative to the maximum capacity of said storage unit.

The respective distance data and respective occupancy data may correspond to the distance data and occupancy data, as described above. Accordingly, the respective distance data may specify a distance along a path for moving the sample container in the automated laboratory system such that a biological sample included in the sample container is processed by the automated laboratory system according to a test order associated with the biological sample, the path comprising the storage unit. The respective occupancy data may comprise information indicative of an occupancy of the storage unit.

Selecting the storage unit may comprise selecting, among the plurality of metrics, a metric which fulfils a set of metric conditions.

For example, the set of metric conditions may comprise either the condition that the metric is the smallest of the plurality of metrics or the condition that the metric is the largest of the plurality of metrics.

The storage unit associated with the metric fulfilling the set of metric conditions may be selected.

According to the present disclosure, a set may comprise one or more elements. For instance, the set of metric conditions may be a collection of one or several distinct conditions. The set of metric conditions, may also comprise a single condition. For example, the set of metric conditions are fulfilled, when all its conditions are fulfilled. In particular, a plurality of items or elements may be a set comprising two or more of said items or elements. For example, a plurality of storage units may comprise two or more storage units.

Each storage unit of the plurality of storage units may comprise or may be associated with a respective recapper for recapping the sample container. In particular, selecting, among the plurality of metrics, the metric may comprise determining whether a second plurality of metrics of the plurality of metrics fulfil the set of metric conditions, and, if the second plurality of metrics fulfil the set of metric conditions, selecting the metric among the second plurality of metrics so that the path comprises the recapper associated with the storage unit associated with the metric.

The recapper of a first storage unit may be connectable, e.g., via the transporting means of the ALS, to the storage space of a second storage unit, such that a sample container recapped by the recapper of the first storage unit can be moved (i.e., "cross-routed") to the storage space of the second storage unit, so that the path of said sample container comprises the recapper of the first storage unit and the storage space of the second storage unit. In particular, when selecting a path of a sample container, a path which does not require cross-routing may be prioritized over a path that requires cross-routing path provided that both paths have (e.g. substantially) the same metric. In particular, two or more path have substantially the same metric if the metrics associated with these paths are comprised in a predefined tolerance range, e.g. within 3%, 5% or 10%. The exemplary percentage numbers may refer to a relative difference between the metrics.

For each storage unit, the occupancy of said storage unit may specify a respective occupancy ratio of the respective recapper. For example, for each storage unit the respective occupancy ratio may be determined based on the actual occupancy of the respective recapper relative to a respective predetermined maximum occupancy capacity of the respective recapper.

In particular, for each storage unit, $S_i$, the metric, $M_i$, may be given by equation (2), wherein, for each storage unit, $S_i$, $L_i$ is a length of the respective path and the inoccupancy ratio, $I_i$, is in particular equal to $(1 - R_i)$ wherein $R_i$ may be the occupancy ratio of the recapper.

In particular, each storage unit may comprise or may be associated with a respective robotic apparatus (e.g., a robotic arm) for moving sample containers in the storage unit e.g., from the respective recapper associated to said storage unit.

For example, for each storage unit, the occupancy of said storage unit may specify a respective occupancy ratio of the respective robotic apparatus. For example, for each storage unit the respective occupancy ratio may be determined based on the actual occupancy of the respective robotic apparatus relative to a respective predetermined maximum occupancy capacity of the respective robotic apparatus.

In particular, for each storage unit, $S_i$, the metric, $M_i$, may be given by equation (2), wherein, for each storage unit, $S_i$, $L_i$ is a length of the respective path and the inoccupancy ratio, $I_i$, is in particular equal to (1 - $R_i$) wherein $R_i$ may be the occupancy ratio of the robotic apparatus.

Exemplarily, the method may further comprise obtaining first path data, the first path data specifying a first path portion (e.g., also referred to as a processing portion of the path) for moving the sample container in the automated laboratory system such that the biological sample included in the sample container is processed by the automated laboratory system according to the test order. In particular, the selected path comprises the first path portion.

For example, in the first path portion the sample container may be preprocessed by the automated laboratory system.

According to a further example, in the path first portion, the biological sample included in the sample container may be processed by the automated laboratory system according to the test order.

In particular, the first path portion may be a route from an input module to a component of the ALS and does not include any storage devices. The first path portion may define the way the sample container is to be transferred through the automated laboratory system from the input module to the component of the ALS so that the biological sample included in the sample container is processed by the automated laboratory system according to the test order associated with the biological sample. Exemplarily, the first path portion does not comprise any recappers.

For example, for each storage unit of the plurality of storage units, the respective path may comprise the first portion. Accordingly, before selecting one of the storage units, the first portion of the path may be determined. Hence, said first portion may be identical for each storage unit.

The first path data may be obtained from an external system or be (pre-) determined by another method. However, the method of the present disclosure may also comprise determining the first path data, e.g. before selecting a storage unit and/or before determining a second path portion (as described below).

For instance, selecting the storage unit may comprise determining second path data specifying a second path portion (herein also referred to as: "storage portion") for moving the sample container in (or to or into) the selected storage unit, wherein the selected path comprises the second path portion.

In particular, determining the second path data may comprise identifying the second path portion. For example, determining the second path data may comprise analyzing relevant information (e.g., the distance data and occupancy data associated with the selected storage unit) and computing the second path portion by identifying one or more components of the ALS that are available and configured to move the sample container from the end point of the first path portion to the selected storage unit, e.g. via the recapper associated with the selected storage unit, and the order according to which said components shall process the sample container. In particular, the second path portion is passed through by said sample container according to the order according to which said components shall process the sample container. For example, the second path portion may be determined such that it is the shortest path that connects the endpoint of the first path portion with the storage unit. The determination may involve or may be implemented as a routine or set of instructions carried out by the computing device.

For example, the second path portion, comprises a recapper specified by the second path data and, hence, the sample container is recapped by said recapper.

In particular, said specified storage portion may be the selected storage device, i.e. as selected by the method of the present disclosure.

The storage unit may be selected and/or the path may be determined when the sample container is in the input module, e.g., the distribution buffer of the input module, e.g., before the sample container leaves the input module.

For instance, selecting the storage unit may comprise determining, for each storage unit, respective second path data specifying a respective second path portion for moving the sample container in said each storage unit, wherein the respective path comprises the second path portion. In particular, for each storage unit of the plurality of storage units, the respective path comprises the second path portion.

The method may further comprise causing, by the computing device, the sample container to be moved within the automated laboratory system along the selected path. Causing the sample container to be moved within the automated laboratory system along the selected path may comprise instructing the ALS to move the sample container, for example by instructing a gripper to put the sample container on tracks and the transportation means to move it according to the path.

The method may further comprise causing, by the computing device, the sample container to be stored in the selected storage unit. Causing the sample container to be stored in the selected storage unit may comprise instructing the ALS to move the sample container in the selected storage unit, for example by instructing a gripper to put the sample container into the storage unit.

The method may further comprise determining, by the computing device and based on useability data (i.e. availability data), the first plurality of storage units from a second plurality of storage units, wherein the useability data comprises information specifying the storage units of the second plurality of storage units which are useable (i.e. available) for storing the sample container, wherein each storage unit of the first plurality of storage units is useable for storing the sample container.

In particular, the method comprises selecting the storage unit among the storage units of the second plurality of storage units that are useable for storing sample containers. For instance, the plurality of storage units, among which a storage unit is selected, i.e., the first plurality of storage units, is limited to the useable storage units.

For example, a storage unit is useable for storing the sample container when: the storage unit is not down (e.g., for maintenance) and is configured, e.g., by a laboratory user, to store sample containers of the type of the sample container that is to be stored.

In particular, the total number of existing storage units (e.g. comprised by or associated with the ALS) may constitute the second plurality of storage units. Among those, the useable storage units may constitute the second plurality of storage units.

The method may further comprise, e.g., after having selected the storage unit:

> obtaining, by the computing device, information specifying that the selected storage unit is not useable any more, and
> after a time interval, selecting another storage unit as destination.

The selection of the other storage unit may be made among the plurality of storage units that are useable at the time point in which the selection of the other storage unit is carried out. In particular, the plurality of storage units from which the other storage unit is selected does not comprise the originally selected storage unit, which is not useable anymore. Exemplarily, the selection of the other storage unit may be carried out by using the method of the present disclosure. The method may comprise selecting, by the computing device, based on distance data and occupancy data, the other storage unit among a plurality of storage units (also referred to as: "third plurality of storage units"), which does not contain the previously selected storage unit, i. The distance data specify a distance along a path for moving the sample container in the automated laboratory system, such that a biological sample included in the sample container is processed (or continued to be processed) by the automated laboratory system according to the test order associated with the biological sample. The path comprises the other storage unit. The occupancy data comprise information indicative of an occupancy of the other storage unit. Hence, another storage unit different from the originally selected one may be selected.

The time interval may for example start at the time point when the information specifying that the selected storage unit is not useable any more has been obtained, or when the non-useability has been detected. The length of the time interval may be predetermined based on an estimate of time needed to move the sample container along the path.

The time interval may depend on the location of the sample container in the ALS at the time point when the information specifying that the selected storage unit is not useable any more has been obtained or when the non-useability has been de detected, Alternatively or in conjunction, the time interval may depend on the cause of non-useability. For example, if the sample container is in the distribution buffer of an input module, the time interval may be equal to about one second, irrespective of the cause of non-useability. For instance, if the sample container is on a track and/or belt, the time interval may be equal to about six seconds if the selected storage unit is not useable because it is down.

Exemplarily the method of the present disclosure may comprise determining, by first computing device, whether, after the time interval, the selected storage unit is still unusable and, if, after the time interval, the selected storage unit is still unusable, selecting another storage unit as destination.

In particular, the method of the present disclosure may further comprise maintaining, for the time interval, the selected storage unit as destination for the sample container.

In general, the method of the present disclosure may be performed by a computing device. Thus, the method may be a computer-implemented method. In case the method steps comprise any actions (for example physical actions) which go beyond a mere data processing operation (for example processing the sample container), the computer-implemented method may further comprise instructions which, when executed by the computing device, cause the ALS (or any elements or components of the ALS) to carry out these actions.

The method of the present disclosure may comprise processing, by the ALS the sample container according to the selected path. In particular, the method of the present disclosure may comprise storing, e.g. by the ALS, the sample container in the selected storage unit.

A second aspect of the present disclosure relates to a computing device (i.e. a data processing system) comprising a processor configured to carry out the method according to the first aspect of the present disclosure. For example, the computing device may further comprise at least one memory storing computer-executable instructions, the computer-executable instructions when executed by the processor cause the computing device and/or its processor to carry out the method according to the present disclosure.

The processor (or processing unit) may be a component of electronic devices that may be responsible for carrying out computational tasks. There may be different types of processing units, each designed for specific purposes. A processing unit may be or may comprise a Central Processing Unit (CPU), Graphics Processing Unit (GPU), Digital Signal Processor (DSP), Field-Programmable Gate Array (FPGA), and/or Application-Specific Integrated Circuit (ASIC).

A third aspect of the present disclosure relates to an automated laboratory system, ALS, comprising the computing device according to the second aspect of the present disclosure, as described above, and/or configured to carry out the method according to the present disclosure.

A fourth aspect of the present disclosure relates to a computer program product comprising instructions which, when the program is executed by a computer system, cause the computer system to carry out the method according to the present disclosure.

The computer system may be the computing device or a part of the computing device according to the second aspect of the present disclosure.

A fifth aspect of the present disclosure relates to a computer-readable medium, e.g., a non-transitory computer-readable medium, comprising instructions which, when executed by a computer system, cause the computer system to carry out the method according to the first aspect of the present disclosure.

In case the method comprise any steps (for example physical) which go beyond a mere data processing operation (for example processing the sample container), the computer program or computer-readable medium may further comprise computer-readable instructions which when executed by a data processing system or by computing device cause the ALS (or any elements or components of the ALS) to carry out these steps.

It is intended that combinations of the above-described elements and those within the specification may be made, except where otherwise contradictory.

It is to be understood that both the foregoing general description and the following detailed description are exemplary and explanatory only, are provided for illustration purposes and are not restrictive for the scope as defined by the accompanied claims.

The accompanying drawings, which are incorporated in and constitute a part of this specification, illustrate examples of the disclosure and together with the description, and serve to support and illustrate the principles thereof.

    Fig. 1 shows a schematic representation of a computing device and a schematic representation of an automated laboratory system.
    Fig. 2 shows a flowchart of an exemplary method.

    Fig. 3a shows a flowchart comprising exemplary steps for selecting a storage unit.
    Fig. 3b shows a flowchart comprising exemplary steps for selecting a storage unit.
    Fig. 4 shows a flowchart comprising exemplary steps for handling a non useability of a selected storage unit.
    Fig. 5a shows a schematic illustration of a recapper.
    Fig. 5b shows a schematic illustration of a recapper.
    Fig. 6 schematically shows a graph 800 representing an exemplary relationship between a metric, an occupancy ratio and a path length.

In the following text, a detailed description of examples will be given with reference to the drawings. Various modifications to the examples may be made. In particular, one or more elements of one example may be combined and used in other examples to form new examples.

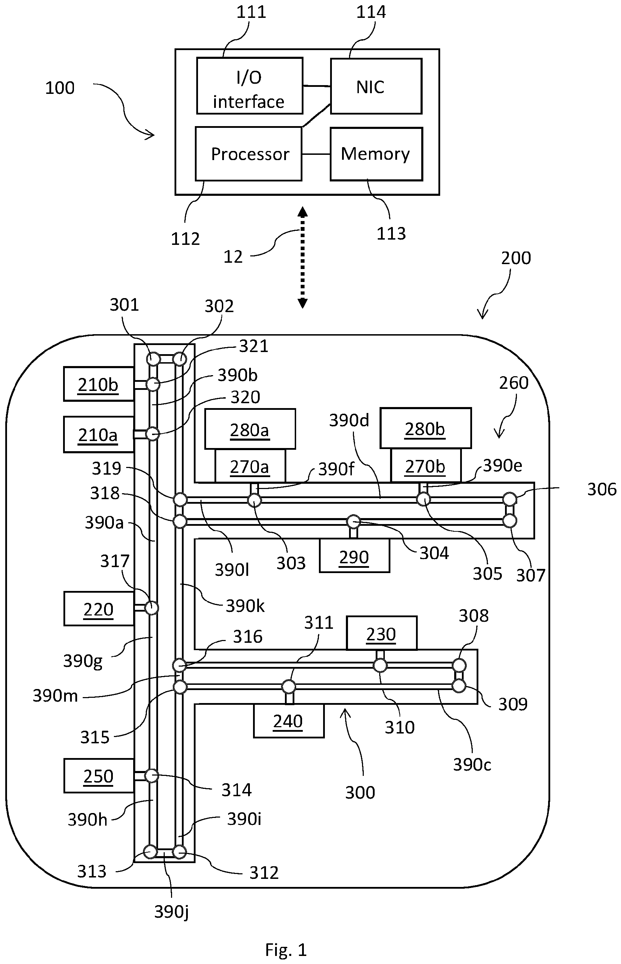

**Fig. 1** shows a schematic representation of a computing device and a schematic representation of an automated laboratory system, ALS, that may be used in some examples of the present disclosure.

The computing device 100 and the automated laboratory system (ALS) 200 may be part of a laboratory. The computing device 100 may include an input/output (I/O) interface 111, a processor 112, a memory 113, and a network interface controller 114. One or more peripheral (input or output) devices (not shown) may be connectable to the input/output interface 111. The peripheral input devices may include one or more of the following: a computer keyboard and/or a pointing device, such as a mouse. The peripheral output devices may include one or more of the following: a terminal, a printer, a disk drive or other storage device, and a video monitor. The processor 112 may include a central processing unit (CPU) and/or a graphics processing unit (GPU), each having one or more processing cores. The memory 113 may include primary memory, such as random access memory (RAM), read-only memory (ROM) or flash memory, and/or secondary memory, such as a solid-state drive or a hard disk drive.

The network interface controller 114 may be connectable to a computer network 12. The ALS 200 may also be connectable to the computer network 12. In some cases, the computing device 100 may be included in (i.e., may be part of) the ALS 200.

The ALS 200 may include a plurality of components. For instance, the ALS 200 comprises a plurality of pre-processing instruments 210a, 210b, 220. In particular, the ALS comprises a plurality, e.g., two, input modules 210a, 210b. Each of the input modules 210a 210b comprises a decapper for decapping sample containers (not shown) and at least a centrifuge for centrifuging sample containers (not shown). Alternatively, each input module of the plurality of input modules 210a, 210b may be associated with a respective centrifuge (not shown) which is mechanically connected with that input module such that sample containers may be transferred from that input module and the respective centrifuge and *vice versa*. The ALS 200 may comprise an aliquoter 220. In some cases, at least an input module of the plurality of modules 210a, 210b may comprise two or more centrifuges and/or at least one input module of the plurality of modules 210a, 210b does not comprise any centrifuge.

Each input module of the plurality of the input modules 210a, 210b may comprise a respective loading area (not shown) for loading sample containers (not shown) in the ALS 200 and for identifying said containers. Moreover, each input module of the plurality of input modules 210a, 210b may comprise a respective distribution buffer (not shown), where sample containers may be positioned before being transferred on a transportation component, e.g., on a track or a belt, to be moved within the ALS 200 for processing.

The ALS 200 comprises one or more processing instruments, i.e., analyzers, 230, 240, 250 for performing tests with a biological sample contained in sample containers (not shown). For example, the ALS 200 comprises a clinical chemistry analyzer 230, an immunoassay analyzer 240 and a hematology analyzer 250. The ALS 200 may also include more or less of these components. For instance, the ALS may comprise a plurality of clinical chemistry analyzers, a plurality of immunoassay analyzers, and/or a plurality of hematology analyzers. The ALS 200 may comprise further analyzers, e.g., one or more urinalysis analyzers (not shown) and/or one or more microbiology analyzers (not shown). The ALS 200 may include oner or more identification and antibiotic susceptibility analyzers, one or more bacteriology analyzers, and/or one or more molecular analyzers.

The ALS 200 comprises a plurality of post-processing instruments 270a, 270b, 280a, 280b, 290. In particular, the ALS may comprise a plurality of, e.g., two, recappers 270a, 270b for recapping sample containers and a plurality of, e.g., two, storage units 280a, 280b for storing tube containers. In particular, each recapper 270a, 270b is associated with a respective storage unit 280a, 280b. For example, the recapper 270a and the recapper 270b are associated with the storage unit 280a and the storage unit 280b, respectively. In particular, a recapper associated with a storage unit is mechanically connected with that storage unit such that sample containers may be transferred from that input module and the respective centrifuge and *vice versa.*

Alternatively, a storage unit of the ALS 200 may comprise a respective recapper associated therewith. A storage unit of the ALS 200 may further comprise a respective storage space (not shown) for storing a plurality of sample containers. The storage space may have several slots (e.g. one slot for one sample container). For example, a sample container may be recapped and then placed in a storage space (i.e., a particular slot) of a storage unit.

The ALS 200 may further comprise an output module 290 where storage units whose processing is incomplete (e.g., because of some processing errors) are diverted e.g., to be handled by a laboratory technician. In particular, the recappers 270a, 270b, the storage units 280a, 280b, and the output module 290 are located in an output region 260 of the ALS 200.

In particular, the recapper 270a is mechanically connected with the storage unit 280b such that sample containers may be transferred from that recapper 270a to that storage unit 280b via the transporting components 390f, 303, 390d, 305, 390e and the recapper 270b. This way, a sample container recapped by the recapper 270a can be cross-routed to the storage unit 280b. For example, the recapper 270b is mechanically connected with the storage unit 280a such that sample containers may be transferred from that recapper 270b to that storage unit 280a via the transporting components 390e, 305, 390d, 305, 390d, 303, 390f and the recapper 270a. This way, a sample container recapped by the recapper 270b can be cross-routed to the storage unit 280a.

The above-mentioned instruments 210a, 210b, 220, 230, 240, 250, 270a, 270b, 280a, 280b, 290 may be (at least indirectly) mechanically connected with each other via a transportation system 300 such that a sample container may be transferred from any of the above-mentioned instruments 210a, 210b, 220, 230, 240, 250, 270a, 270b, 280a, 280b, 290 to any other instrument 210a, 210b, 220, 230, 240, 250, 270a, 270b, 280a, 280b, 290 by means of the transportation system 300.

Specifically, the transportation system 300 includes a plurality of transport components 301-321, 390a, 390b, 390c, 390d, 390e, 390f. In particular, the transport system may comprise a plurality of diverters 301-321 and a plurality of tracks and/or belts 390a, 390b, 390c, 390d, 390e, 390f. A diverter is in particular a mechanical device which allows to transfer a sample container from a track and/or belt to another track and/or belt. In particular, a diverter, depending on the path that a sample container shall follow, allow for changing the direction of motion of said sample container.

The transportation system 300 comprises a plurality of tracks and/or belts 390a, 390b, 390c, 390d, 390e, 390f for transporting sample containers e.g., in container carriers (not shown). In Fig. 1 each white rectangle comprised between two diverters and each white rectangle comprised between a diverter and an instrument. For instance, the track and/or belt 390c is comprised between the diverters 309 and 310, the track and/or belt 390d is comprised between the diverters 303 and 305, the track and/or belt 390b is comprised between the diverters 320 and 321, and the track and/or belt 390a is comprised between the diverters 320 and 317. Moreover, the track and/or belt 390b is comprised between the diverter 303 and the recapper 270a, and the track and/or belt 390e is comprised between the diverter 305 and the recapper 270b. In particular, the transport components 301-321, 390a, 390b, 390c, 390d, 390e, 390f are configured to move sample containers within the ALS 200. The transport components are only schematically indicated in fig. 1. The transportation system 300 may comprise more or less transport components. For example, the transportation system 300 may comprise one or more robotic grippers (not shown) and/or one or more container carriers (not shown).

The transportation system 300 may further comprise at least one buffer region (not shown) configured to temporarily store one or more of sample containers. The buffer region may be mechanically connected with any one of the instruments 210a, 210b, 220, 230, 240, 250, 270a, 270b, 280a, 280b, 290 of the ALS 200 such that a sample container may be transferred from any of the above-mentioned instruments to the buffer region by means of the transport components of the transportation system 300.

A sample container may thus be transferred along a determined path from an input module 210a, 210b to one of the storage units 280a, 280b, in particular along a determined path (as explained in more detail below). In particular, a path comprises one or more of the instruments 220, 230, 240, 250, and a plurality of the transportation components 301-321, 390a, 390b, 390c, 390d, 390e, 390f. The plurality of transportation components allows the sample container to travel in the instruments comprised in said path so that said instruments may pre-process, post-process and/or process the biological sample contained in the sample container. Alternatively, or additionally, the path may comprise a plurality of transportation components that allows the sample container to travel in the vicinity of one or more instruments that are not comprised in the route, so that said instruments are able to retrieve and at least a portion of the biological sample contained in the sample container, and pre-process, process and/or post process said portion..

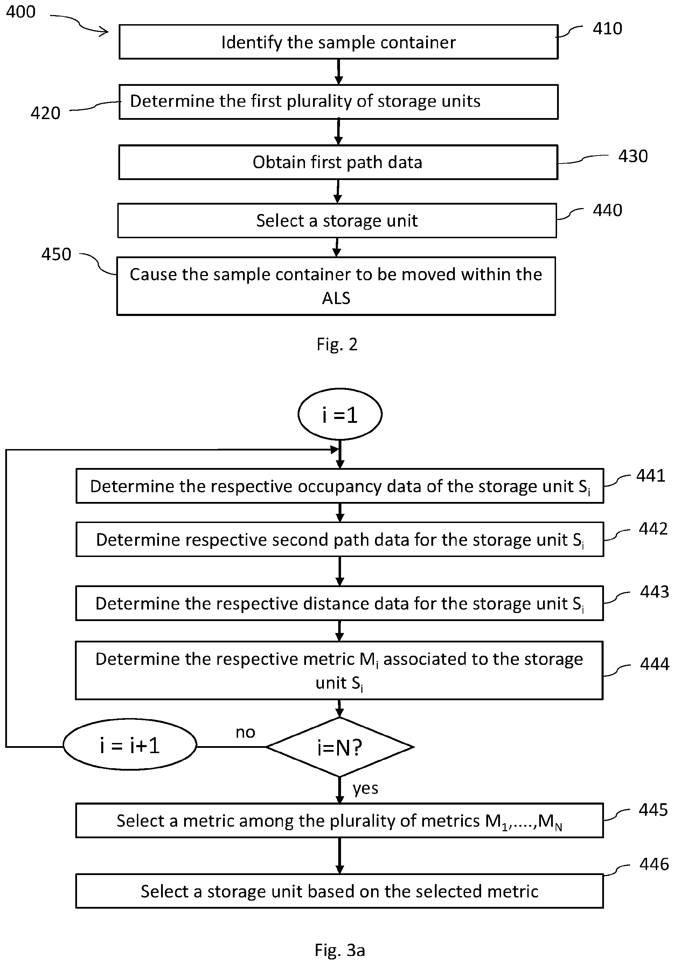

**Fig. 2** shows a flowchart of an exemplary method.

The method may be carried out by a computing device, i.e. it may be a computer implemented method, and in particular by the computing device schematically depicted in fig. 1 and described above.

Optional step 410 of the method may comprise identifying a sample container introduced in an input module, say the input module 210a, of the ALS by a laboratory technician. In particular, upon receipt of the sample container, a barcode reader (not shown) of the input module reads identification data comprised in a barcode attached to the sample container. In particular, the identification data comprise information that uniquely identifies the sample container and allows the computing device 100 to obtain, e.g., by querying the LIS (not shown in fig. 1), the test order associated with the sample container. The ALS 200 provides the identification data to the computing device 100, which identifies the sample container by using said data. In particular, the computing device 100 obtains the test order by querying the LIS. In an exemplary case, the test order may specify one or more hematology tests.

At optional step 420 it may be determined, based on useability data, the first plurality of storage units. In particular, the first plurality of storage units comprises the storage units of the ALS 200 that are useable for storing sample containers. In particular, a storage unit may be unusable for storing because at least one of its elements (e.g. a robotic arm) is broken or it is under maintenance. In particular, the computing device 100 may obtain, e.g., receive, the useability data from the ALS 200. The useability data comprises information specifying, for each storage unit 280a, 280b of the ALS 200, whether said each storage unit is useable or not.

In particular, at optional step 420, the computing device 100 determines the storage units of the ALS 200 that are useable by accessing the useability data. In the exemplary case, both the storage units 280a, 280b of the ALS 200 are useable and, hence, in this exemplary case, the first plurality of storage unit consists of the storage unit 280a and the storage unit 280b.

At optional step 430, the computing device 100 obtains first path data. The first path data specifies a first path portion for moving the sample container in the automated laboratory system such that the biological sample included in the sample container is processed by the automated laboratory system according to the test order.

In particular, at optional step 430, the computing device 100 may determine, by using the test order, the pre-processing, processing and post-processing steps (herein also collectively referred to as: "processing steps") to be performed on the biological sample and/or on the sample container including the biological sample to carry out the tests in the test order. For instance, a processing step may be the step of decapping the sample container carried out by the decapper of one of the input modules 210a, 210b. For example, a processing step may be the step of transporting the sample container from an instrument 210a, 210b, 220, 230, 240, 250, 270a, 270b, 280a, 280b, 290 to another instrument 210a, 210b, 220, 230, 240, 250, 270a, 270b, 280a, 280b, 290 and may be carried out by a plurality of transportation components 301-321, 390a, 390b, 390c, 390d, 390e, 390f. For example, a processing step may be the step of performing a clinical test on the biological sample and may be carried out by an analyzer 230, 240, 250. A processing step may be the step of recapping the sample container and may be carried out by one of the recappers 270a, 270b of the ALS 200. Another processing step is the step of storing the sample container in one of the storage units 280a, 280b.

At optional step 430, the computing device 100 may access data specifying the instruments included in the ALS 200 and determine the instruments 220, 230, 240, 250 that can carry out the aforementioned steps. The computing device may obtain, e.g., from the ALS 200, data specifying the availability and the workload of the instruments 220, 230, 240, 250 and use said data to generate the first path data, i.e., the first path portion, e.g.,

by selecting the shortest path that does not comprise the recappers 270a, 270b and the storage units 280a, 280b and that allows all the aforementioned pre-processing, processing and post-processing steps to be carried out reliably.

In particular, path data specifies a respective path portion for a sample container, e.g., by providing information specifying an ordered list of components 210a, 210b, 220, 230, 240, 250, 270a, 270b, 280a, 280b, 290, 301-321, 390a, 390b, 390c, 390d, 390e, 390f of the ALS 200. In particular, the ordering of the list reflects the chronological order according to which the components shall be reached by said sample container. Hence, the ordered list may be a temporal list.

In particular, the path data may identify a plurality of instruments 210a, 210b, 220, 230, 240, 250, 270a, 270b, 280a, 280b, 290 within the automated laboratory system 200 and may indicate a path through the plurality of components that the sample container should follow.

For example, the path data may specify an order according to which the sample should be handled by the components of the automated laboratory system, i.e., a temporal sequence of processing steps. Some processing steps may be carried out sequentially, one after the other, other processing steps may be carried out with at least a partial time overlap, yet other processing steps may be carried out at any point in time and may, thus, not be included in the temporal sequence (while still being part of the path). Accordingly, the path data may comprise an ordered list of processing steps that each component of the automated laboratory system should carry out.

In the exemplary case, the test order specifies one or more hematology tests. The computing device 100 accesses the data specifying the instruments included in the ALS 200 and determines that the hematology analyser 250 is configured and available for carrying out said hematology tests. The computing device 100 determines the first path portion as the shortest path that starts from the input module 210a, that comprises the hematology analyzer 250 and that finishes at the diverter 319, i.e., at the entrance point of the output area 260 of the ALS 200. In particular, the first path portion is specified by the following ordered list of components of the ALS 200: 210a - 320 - 390a - 317 - 390g - 314 - track and/or belt between 314 and 250 - 250 - track and/or belt between 314 and 250 - 314 - 390h - 313 - 390j - 312 - 390i - 315 - 390m- 316 - 390k - 318 - track and/or belt between 318 and 319 - 319.

Alternatively, the first path data specifying the first portion may be obtained or determined from a database or a lookup table based on the identification data.

Steps 420 and 430 may be carried out simultaneously or sequentially in any order.

At step 440, the computing device 100 selects the storage unit for storing the sample container.

In some cases, the selection of the storage unit is carried out as schematically shown in **fig.3a** and described below. In particular, for each storage unit of the N storage units in the first plurality of storage units, the computing device 100 carries out the steps 441 to 444.

With reference to fig. 3a, for each storage unit, $S_i$, (i = 1,.., N) of the first plurality of storage units, the computing device 100 determines 441 the respective occupancy data of $S_i$. In particular, the respective occupancy data may consist of the respective occupancy ratio, $R_i$, of the respective recapper, $C_i$, associated with said storage unit, $S_i$. In particular, the occupancy ratio $R_i$ is determined by receiving, e.g., from the ALS 200, information specifying the number of sample containers, $K_i$, which are located in the belt and/or track mechanically connected with the respective recapper $C_i$. For example, if the recapper $C_i$ is the recapper 270a, the information received from the ALS 200 specifies the number of sample containers in the track and/or belt 390f. If the recapper $C_i$ is the recapper 270b, the information received from the ALS 200 specifies the number of sample containers in the track and/or belt 390e. The computing device 100 may then determine $R_i$ by dividing $K_i$ by the predetermined maximum occupancy capacity $U_i$ of the belt and/or track mechanically connected with the respective recapper $C_i$. In particular, the maximum occupancy may be equal to the predefined maximum amount of sample containers that the respective belt and/or track mechanically connected with the respective recapper $C_i$ can accommodate. Alternatively, the maximum occupancy may be equal to 70% or 80% of the predefined maximum amount of sample containers that the respective belt and/or track mechanically connected with the respective recapper $C_i$ can accommodate.

In the exemplary case, for instance, N=2, the storage unit 280a corresponds to $S_1$ and the storage unit 280b corresponds to $S_2$ (hence $C_1$ and $C_2$ corresponds to 270a and 270b, respectively).

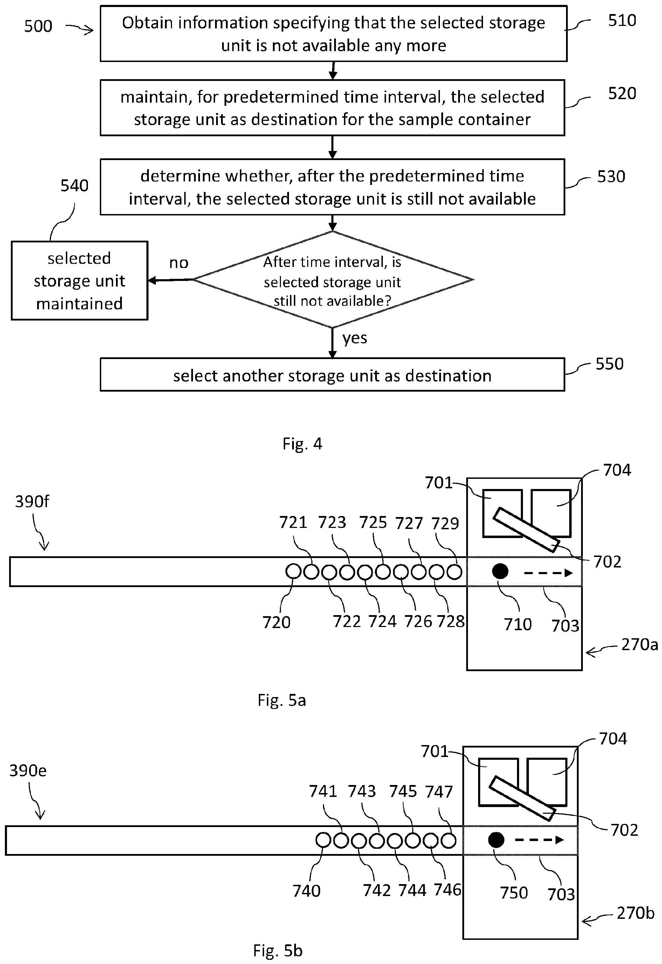

**Fig. 5a** shows a schematic illustration of the recapper 270a and the track and/or belt 390f comprised in the output region 260 (cf. fig. 2). The recapper 270a comprises a recapping device 701 configured to close or seal a sample container (e.g., sample container 710) by means of a cap. In particular the recapping device 701 comprise a robotic arm 702 configured to pick a cap from a cap container 704 of the recapper 270a and to apply the cap to the opening of a sample container (e.g., of the sample container 710). The recapper comprises a track and/or belt 703 to move a sample container from the track and/or belt 390f and a recapping position (e.g., the position at which the sample container 710 is located) and from the recapping position to the storage device 280a. The computing device 100 determines the occupancy ration $R_1$ by dividing $K_1$ by the predetermined maximum occupancy capacity $U_1$ of the belt and/or track 390f which, in this case, is equal to the to the predefined maximum amount of sample containers that the belt and/or track 390f can accommodate ($U_1$=25). the number $K_1$ is the number of sample containers which are located in the belt and/or track 390f. In this case, $K_1$=10, as ten sample containers 720-729 are located on the belt and/or track 390f. Hence, in this case, $R_1 = 0.4$.

The actual occupancy, $K_1$, of the recapper 270a may be determined by using the data provided by a first sample container detector (not shown) and a second sample container detector (not shown). In particular, the first sample container detector is arranged with respect to track and/or belt 390f such that said detector detects sample containers entering the track and/or belt 390f. The second sample container detector is arranged with respect to the recapping device 701 such that said detector detects sample containers that leave the recapper and/or are recapped by the recapper and, hence, are about to leave the recapper.

The recapper device 701 may take a predefined time for processing (i.e. recapping) a sample container. Accordingly, a recapping rate can be determined, i.e. the number of sample containers which can be processed by the recapping device. Therefore, the number of "waiting" sample containers in the track and/or belt 390f may change depending on the rate of arriving sample containers in relation to the processing rate of the recapper device. For this reason, the occupancy, i.e. the occupancy ratio, may vary over the time.

**Fig. 5b** shows a schematic illustration of the recapper 270b and the track and/or belt 390e comprised in the output region 260 (cf. fig. 2). The recapper 270b may be identical to the recapper 270a as shown in figs. 2 and 5a and described above. The computing device 100 determines the occupancy ratio $R_2$ by dividing $K_2$ by the predetermined maximum occupancy capacity $U_2$ of the belt and/or track 390e which, in this case, is equal to the to the predefined maximum amount of sample containers that the belt and/or track 390e can accommodate ($U_2 = 25$). The number $K_2$ is the number of sample containers which are located in the belt and/or track 390e. In this case, $K_2 = 8$, as eight sample containers 740-747 are located on the belt and/or track 390e. Hence, in this case, $R_2 = 0.32$.

With reference to fig. 3a, for each storage unit, $S_i$, ($i = 1,.., N$) of the first plurality of storage units, the computing device 100 determines 442 second path data specifying a respective second path portion for moving the sample container in the storage unit $S_i$ e.g., via the recapper $C_i$. in particular, the second path data are determined by determining the respective second path portion, as the shortest path that connects the endpoint of the first path portion with the storage unit $S_i$ and comprises the respective recapper $C_i$, if available. If the respective recapper $C_i$ is not useable, the respective second path portion may be the shortest path that connects the endpoint of the first path portion with the storage unit $S_i$ and comprises the recapper of the ALS 200 with the smallest occupancy ratio. In this case, the second path portion comprises cross routing.

In the exemplary case, for instance, the second path portion associated with the storage unit 280a is given by: 390l - 303 - 390f - 270a - 280a. The second path portion associated with the storage unit 280b is given by: 390l - 303 - 390d -305 - 390e - 270b - 280b.

For each storage unit, $S_i$, ($i = 1,.., N$) of the first plurality of storage units, the computing device 100 determines 443 the respective distance data for the storage unit $S_i$. In particular, the computing device determines a respective path associated with the storage unit $S_i$, wherein the respective path is the union of the first path portion and the respective second path portion associated with the storage unit $S_i$. In particular the respective distance data for the storage unit $S_i$ may be the length, $L_i$, of the respective path, e.g., expressed in a given unit of measure, e.g., centimeters or meters. In particular, the computing device 100 may determine the length $L_i$, by using the first path data, the respective second path data and the length of the belts and/or tracks included in the first path portion and the respective path portion. In some cases, the length, $L_i$, of the respective path may be expressed as the total number of pre-processing instruments, analyzers, post-processing instruments and diverters, included in the respective path.

In the exemplary case, for instance, the path associated with the storage unit 280a is given by 210a - 320 - 390a - 317 - 390g - 314 - track and/or belt between 314 and 250 - 250 - track and/or belt between 314 and 250 - 314 - 390h - 313 - 390j - 312 - 390i - 315 - 390m- 316 - 390k - 318 - track and/or belt between 318 and 319 - 319 - 390l - 303 - 390f - 270a - 280a, and its length, $L_1$, may be equal to 22 units of measure. The path associated with the storage unit 280b is given by 210a - 320 - 390a - 317 - 390g - 314 - track and/or belt between 314 and 250 - 250 - track and/or belt between 314 and 250 - 314 - 390h - 313 - 390j - 312 - 390i - 315 - 390m- 316 - 390k - 318 - track and/or belt between 318 and 319 - 319 - 390l - 303 - 390d -305 - 390e - 270b - 280b and its length, $L_2$, may be equal to 25 units of measure.

In particular, if $L_1$ and $L_2$ are expressed as the total number of pre-processing instruments, analyzers, post-processing instruments and diverters, included in the respective paths, the following relations hold $L_1 = 15$ and $L_2 = 16$.

For each storage unit, $S_i$, ($i = 1,.., N$) of the first plurality of storage units, the computing device 100 determines 444 the respective metric $M_i$, by using $R_i$, $L_i$ and equation (2) described above.

In the exemplary case, for instance, the metric, $M_1$, associated with the storage unit 280a is equal to about 61.1 and the metric, $M_2$, associated with the storage unit 280b is equal to about 54.1.

At step 445, the computing device 100 selects, among the first plurality of metrics, i.e., among the metrics $M_1, ..., M_N$, a metric that fulfills a set of metric conditions. For example, the set of metric conditions may comprise either the condition that the metric is the smallest of the plurality of metrics or the condition that the metric is the largest of the plurality of metrics. If only a metric fulfills the set of metric conditions, the storage unit associated with that only metric is then selected 446 as the storage unit for storing the sample container. In particular, if more than one metric fulfills the set of metric conditions, the computing device 100 selects e.g. randomly one metric of the metrics fulfilling the set of metric conditions and selects 446 the storage unit associated with that metric as the storage unit for storing the sample container. An alternative selection in presence of more than one metrics fulfilling the set of metric conditions.

In the exemplary case, for instance, the set of metric conditions consists of the condition that the metric is the smallest of the plurality of metrics $M_1$ and $M_2$, and hence, the metric selected is $M_2$. Thus, the storage unit 280b is selected as the storage unit for storing the sample container.

With reference to fig. 2, at optional step 450, the computing device 100 causes the sample container to be moved within the ALS 200 along the selected path. In particular, at optional step 450, the computing device 100 instructs the ALS 200 to move the sample container along the selected path.

In the exemplary case, for instance, the computing device 100 causes the sample container to be moved within the ALS 200 along the path associated with the storage unit 280b, i.e., the path: 210a - 320 - 390a - 317 - 390g - 314 - track and/or belt between 314 and 250 - 250 - track and/or belt between 314 and 250 - 314 - 390h - 313 - 390j - 312 - 390i - 315 - 390m - 316 - 390k - 318 - track and/or belt between 318 and 319 - 319 - 390l - 303 - 390d -305 - 390e - 270b - 280b.

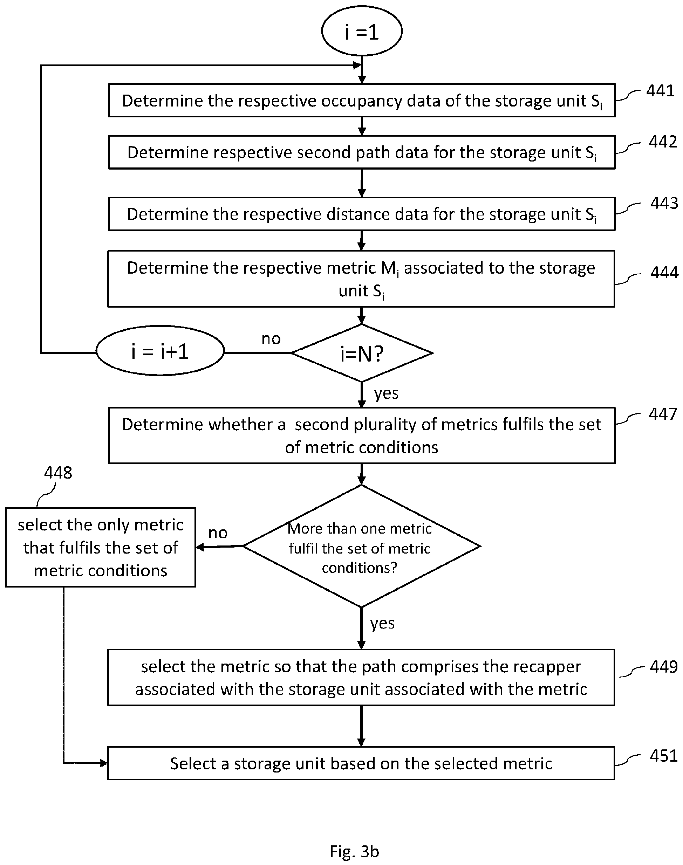

In some other cases, with reference to fig. 2, selecting 440 the storage unit is carried out as schematically shown in **fig. 3b** and described below. With reference to fig. 3b, the computing device 100 carries out the steps 441 to 444 as disclosed above in the description of fig. 3a. At step 447, the computing device 100 determine whether a second plurality of the metrics $M_1,...,M_N$ fulfills the set of metric conditions. In particular, the computing device 100 checks whether more than one metric is the smallest among the metrics $M_1,...,M_N$. If only one metric fulfills the set of metric conditions, that only metric is selected 446 and at step 451, the computing device 100 selects 451 the storage unit associated with that only metric as the storage unit for storing the sample container. If a second plurality of the metrics $M_1,...,M_N$ fulfills the set of metric conditions, the computing device 100 selects the metric so that the path comprises the recapper associated with the storage unit associated with the metric and selects 451 the storage unit associated with that metric as the storage unit for storing the sample container.

Hence, in this case, the path associated with the selected storage unit does not involve any cross-routings.

In other words, in this case, a path without cross-routing (and its associated storage unit) is prioritized over a path with cross-routing, if both paths have the same metric. In this case, a storage unit involving cross-routing may only be favorable if it leads to a (e.g. substantially) better metric.

**Fig. 4** shows a flowchart 500 comprising exemplary steps for handling a non useability of a selected storage unit.

After a storage unit is selected for storing the sample container, e.g. according to the flowchart schematically shown in fig. 2 and described above, at step 510 the computing device 100 obtains information specifying that the storage unit is not useable any more. In particular, said information may be provided by the ALS 200, e.g., upon detection of a malfunction of the selected storage unit. For instance, the recapper of the storage unit may have a defect or may have no caps any more. In particular, the ALS 200 may send to the computing device 100 the information specifying that the storage unit is not useable any more. Hence, obtaining said information may be carried out by receiving said information.

At step 530 the selected storage unit is maintained as destination for the sample container for time interval t. In particular, at step 530, the computing device 100 monitors the time elapsed from the receipt of the information specifying that the storage unit is not useable any more. When the time interval t has expired, the computing device 100 determines whether the the selected storage unit is still not useable. The determination 530 may comprise querying the ALS 200 about the useability status of the selected storage unit. If, after the time interval t has expired, the selected storage unit is useable, the selected storage unit is maintained 540 as destination for the sample container.

If, after the time interval t has expired, the selected storage unit is still not useable, another storage unit is selected as destination in step 550. The selection may be made among the plurality of storage units that are currently useable (i.e. another storage unit different from the originally selected one) by using the steps of the flowchart depicted in fig. 2.

The flowchart 500 may have the advantage of making the whole process more robust against detected malfunctions, as the originally selected storage unit is temporarily maintained as destination for the sample container (which is already under process). It is namely possible that the storage unit can anyway still store the sample container, as e.g. the malfunction detection is incorrect or the respective error message is anticipative, i.e. only relevant for any future sample container (i.e. to be processed in future).

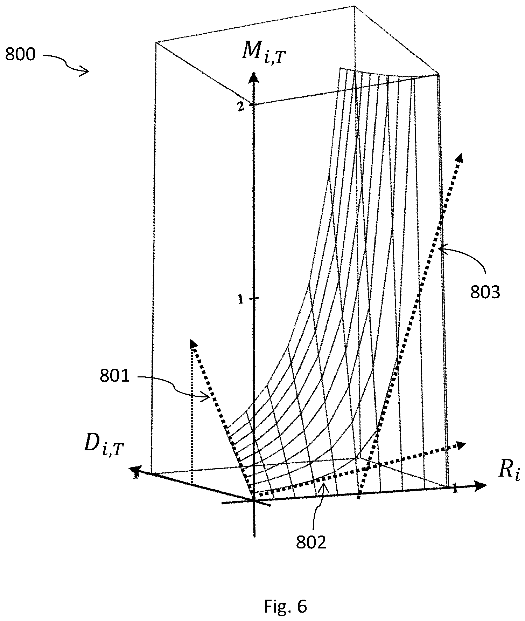

**Fig. 6** schematically shows a graph 800 depicting the functional dependency of the metric Mi from the length Li and the occupancy ratio Ri, as defined in equation (2) above.

The rationale for this exemplary metric (as defined in equation (2)) is that for a relatively small occupancy ratio $R_i$, e.g., $R_i$ close to 0, the length $L_i$ has a relatively large impact on the metric $M_i$. This is schematically illustrated by the relatively high gradient (i.e. slope) of line 801 (representing the relatively strong impact of an increasing length $L_i$ on the metric $M_i$) in relation to the gradient of line 802 (representing the relatively low impact of an increasing occupancy ratio $R_i$ when $R_i$ is still relatively close to 0). This way, if two storage units have a relatively small value of the occupancy ratio the storage unit associate with the shortest path may be associated with the smallest metric even if its occupancy ratio is slightly higher compared to the other storage unit.

At a relatively high occupancy ratio $R_i$, e.g., $R_i$ close to 1, the path length $L_i$ may have a relatively small influence on the metric $M_i$ and the occupancy ratio $R_i$ has a relatively large one, as schematically illustrated by the relatively high gradient (i.e. slope) of line 803 (representing the relatively strong impact of an increasing occupancy ratio $R_i$ on the metric $M_i$ when $R_i$ is becoming closer to 1). This way, if two storage units are associated with a relatively high occupancy ratio, the storage unit associated with the slightly lower occupancy ratio will be associate to the smallest metric, even if its associated path length is greater than path length associated with the other storage unit. This behavior will avoid, at least in part, congestions in correspondence to the storage units.

Throughout the description, including the claims, the term "comprising a" should be understood as being synonymous with "comprising at least one" unless otherwise stated. In addition, any range set forth in the description, including the claims should be understood as including its end value(s) unless otherwise stated. Specific values for described elements should be understood to be within accepted manufacturing or industry tolerances known to one of skill in the art, and any use of the terms "substantially" and/or "approximately" and/or "generally" should be understood to mean falling within such accepted tolerances.

Although the present disclosure herein has been described with reference to particular examples, it is to be understood that these examples are merely illustrative of the principles and applications of the present disclosure.

It is intended that the specification and examples be considered as exemplary only, with a true scope of the disclosure being indicated by the following claims.

A reference herein to a patent document or any other matter identified as prior art, is not to be taken as an admission that the document or other matter was known or that the information it contains was part of the common general knowledge as at the priority date of any of the claims.

## Claims

1. A method comprising:

   • selecting, by a computing device based on distance data and occupancy data, a storage unit among a plurality of storage units for storing a sample container processed by an automated laboratory system,
   wherein the distance data specify a distance along a path for moving the sample container in the automated laboratory system such that a biological sample included in the sample container is processed by the automated laboratory system according to a test order associated with the biological sample, the path comprising the storage unit, and
   wherein the occupancy data comprise information indicative of an occupancy of the storage unit.

2. The method according to the preceding claim, wherein selecting the storage unit comprises at least one of:

   determining a metric based on the distance data and the occupancy data, the metric being associated with the storage unit, and
   determining whether the metric fulfills a first set of selection criteria, wherein, optionally, the first set of selection criteria comprises one or more of: a criterium that the metric is lower than a first predetermined threshold, a criterium that the metric is lower than or equal to the first predetermined threshold, a criterium that the metric is greater than a second predetermined threshold, a criterium that the metric is greater than or equal to the second predetermined threshold.

3. The method according to any one of the preceding claims, wherein
   the distance along the path for moving the sample container is specified by at least one of:

   a length of the path,
   a number of components of the automated laboratory system that are included in the path, and
   an estimate of a time needed to move the sample container along the path.

4. The method according to any one of the preceding claims, wherein the occupancy of the storage unit is specified by a fill ratio of the storage unit, and wherein, optionally, the fill ratio is determined based on a number of sample containers comprised in the storage unit relative to a maximum number of sample containers storable in the storage unit.

5. The method according to any one of the preceding claims, wherein the storage unit comprises or is associated with a respective recapper for recapping the sample container, wherein the occupancy of the storage unit specifies an occupancy ratio of the recapper and wherein, optionally, the occupancy ratio is determined based on the actual occupancy of the recapper relative to a predetermined maximum occupancy capacity of the recapper, and/or wherein the storage unit comprises or is associated with a respective robotic apparatus for moving sample containers in the storage unit from the recapper associated to said storage unit, wherein occupancy of the storage unit is defined as an occupancy ratio of the respective robotic apparatus, said occupancy ratio being optionally determined based on the actual occupancy of the robotic apparatus relative to a predetermined maximum occupancy capacity of the robotic apparatus.

6. The method of any of the previous claims, wherein selecting the storage unit comprises:

   ○ determining, for each storage unit of the plurality of storage units, and based on respective distance data and respective occupancy data, a respective metric associated with said each storage unit, thereby obtaining a plurality of metrics, wherein the respective distance data specify a respective distance along a respective path in the automated laboratory system, the respective path comprising said each storage unit, and wherein the respective occupancy data comprise information indicative of a respective occupancy of said each storage unit, and wherein selecting the storage unit is based on the plurality of metrics, and/or selecting the storage unit comprises comparing the plurality of metrics.

7. The method of the preceding claim, wherein selecting the storage unit comprises:

   ○ selecting, among the plurality of metrics, a metric which fulfils a set of metric conditions, the set of metric conditions comprising either the condition that the metric is the smallest of the plurality of metrics or the condition that the metric is the largest of the plurality of metrics, wherein the storage unit associated with the metric is selected.

8. The method of the previous claim, wherein each storage unit of the plurality of storage units comprises or is associated with a respective recapper for recapping the sample container, wherein selecting, among the plurality of metrics, the metric comprises determining whether a second plurality of metrics of the plurality of metrics fulfil the set of metric conditions, and, if the second plurality of metrics fulfils the set of metric conditions, selecting the metric among the second plurality of metrics so that the path comprises the recapper associated with the storage unit associated with the metric.

9. The method according to any one of the preceding claims, further comprising obtaining first path data specifying a first path portion for moving the sample container in the automated laboratory system such that the biological sample included in the sample container is processed by the automated laboratory system according to the test order, wherein the path comprises the first path portion.

10. The method according to any one of the preceding claims, wherein selecting the storage unit comprises determining second path data specifying a second path portion for moving the sample container in the selected storage unit, wherein the path comprises the second path portion.

11. The method according to any one of the preceding claims, further comprising determining, by the computing device based on useability data, the plurality of storage units from a second plurality of storage units, wherein the useability data comprises information specifying the storage units of the second plurality of storage units which are useable for storing sample containers, wherein each storage unit of the first plurality of storage units is useable for storing sample containers.

12. The method according any one of the preceding claims, further comprising obtaining, by the computing device, information specifying that the storage unit is not useable any more, maintaining, for a time interval, the selected storage unit as destination for the sample container, and after a time interval, selecting another storage unit as destination.

13. A computing device comprising a processor configured to carry out the method according to any one of the preceding claims.

14. A computer program product comprising instructions which, when the program is executed by a computer system, cause the computer system to carry out the method according to any one of the claims 1 to 12.

15. A computer-readable medium comprising instructions which, when executed by a computer system, cause the computer system to carry out the method according to any one of the claims 1 to 12.

Fig. 1

400

| Identify the sample container | 410 |

| Determine the first plurality of storage units | 420 |

| Obtain first path data | 430 |

| Select a storage unit | 440 |

| Cause the sample container to be moved within the ALS | 450 |

Fig. 2

$i = 1$

| Determine the respective occupancy data of the storage unit $S_i$ | 441 |

| Determine respective second path data for the storage unit $S_i$ | 442 |

| Determine the respective distance data for the storage unit $S_i$ | 443 |

| Determine the respective metric $M_i$ associated to the storage unit $S_i$ | 444 |

$i = i+1$ ← no — $i=N?$

yes

| Select a metric among the plurality of metrics $M_1,....,M_N$ | 445 |

| Select a storage unit based on the selected metric | 446 |

Fig. 3a

Fig. 3b

500 →

| Obtain information specifying that the selected storage unit is not available any more | ⌐ 510 |

↓

| maintain, for predetermined time interval, the selected storage unit as destination for the sample container | ⌐ 520 |

↓

| determine whether, after the predetermined time interval, the selected storage unit is still not available | ⌐ 530 |

↓

540

| selected storage unit maintained | ← no — After time interval, is selected storage unit still not available? |

↓ yes

| select another storage unit as destination | ⌐ 550 |

Fig. 4

390f

721 723 725 727 729

704
701
702
710 703
270a

720  722 724 726 728

Fig. 5a

390e

741 743 745 747

704
701
702
750 703
270b

740  742 744 746

Fig. 5b

Fig. 6

European
Patent Office

Europäisches
Patentamt

European
Patent Office

Office européen
des brevets

## EUROPEAN SEARCH REPORT

**Application Number**

EP 24 18 5772

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 4 361 638 A1 (BECKMAN COULTER INC [US]) 1 May 2024 (2024-05-01) * paragraph [0006] - paragraph [0118] * ----- | 1-15 | INV. G16H10/40 |
| A | US 2020/174030 A1 (GOEMANN-THOSS WOLFGANG [DE] ET AL) 4 June 2020 (2020-06-04) * paragraph [0011] - paragraph [0229] * ----- | 1-15 | |
| A | CN 109 313 206 A (ANDREW ALLIANCE S A) 5 February 2019 (2019-02-05) * the whole document * ----- | 1-15 | |

**TECHNICAL FIELDS SEARCHED (IPC)**

G16H

The present search report has been drawn up for all claims

| Place of search | Date of completion of the search | Examiner |
|---|---|---|
| Munich | 11 December 2024 | Rinelli, Pietro |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

  .................................................................................

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 24 18 5772

11-12-2024

| Patent document cited in search report | | | Publication date | Patent family member(s) | | Publication date |
|---|---|---|---|---|---|---|
| EP 4361638 | A1 | | 01-05-2024 | EP | 4361638 A1 | 01-05-2024 |
| | | | | WO | 2024089613 A1 | 02-05-2024 |
| US 2020174030 | A1 | | 04-06-2020 | CN | 105745545 A | 06-07-2016 |
| | | | | EP | 2857843 A1 | 08-04-2015 |
| | | | | EP | 3055697 A1 | 17-08-2016 |
| | | | | US | 2015104796 A1 | 16-04-2015 |
| | | | | US | 2020174030 A1 | 04-06-2020 |
| | | | | WO | 2015051906 A1 | 16-04-2015 |
| CN 109313206 | A | | 05-02-2019 | CN | 109313206 A | 05-02-2019 |
| | | | | EP | 3443358 A1 | 20-02-2019 |
| | | | | JP | 2019514003 A | 30-05-2019 |
| | | | | KR | 20180132142 A | 11-12-2018 |
| | | | | US | 2017299619 A1 | 19-10-2017 |
| | | | | WO | 2017178890 A1 | 19-10-2017 |